# FASCICULE DE BREVET EUROPEEN

(11) **EP 2 136 723 B1**
(45) Date de publication et mention de la délivrance du brevet: **18.08.2010**
(21) Numéro de dépôt: 08788202.3
(22) Date de dépôt: 18.04.2008
(51) Int. Cl.: A61B 17/70, A61B 17/68

(54) **SYSTÈME D'OSTÉOSYNTHÈSE POUR RELIER AU MOINS DEUX VERTÈBRES**
OSTEOSYNTHESESYSTEM ZUR VERBINDUNG MINDESTENS ZWEIER WIRBEL
OSTEOSYNTHESIS SYSTEM FOR CONNECTING AT LEAST TWO VERTEBRAE

(30) Priorité: 19.04.2007 FR 0754575
(43) Date de publication de la demande: 30.12.2009
(73) Titulaire: Ceria - Conception Etudes Realisation D'implants Et D'Ancillaires, 37360 Semblancay (FR); Limito, Jean-François, 31130 Balma (FR); Renaud, Christian, 81160 Arthes (FR)
(72) Inventeur: RENAUD, Christian, F-81160 Arthes (FR)
(74) Mandataire: Blot, Philippe Robert Emile
(86) Numéro de dépôt international: PCT/FR2008/050700
(87) Numéro de publication internationale: WO 2008/145914

(56) Documents cités:
- EP-A- 1 206 924
- EP-A- 1 743 587
- WO-A-98/37825
- US-A- 4 716 893
- US-A1- 2006 167 459
- US-A1- 2006 241 593

## Description

La présente invention concerne un système d'ostéosynthèse pour relier au moins deux vertèbres, du type comportant des éléments de fixation dans chacune des vertèbres.

On connaît déjà des systèmes d'ostéosynthèse de vertèbres par voie postérieure comprenant une plaque percée de deux trous à travers lesquels sont insérées deux vis de fixation constituant un ensemble de fixation et destinées à venir chacune en prise dans une vertèbre. Deux vertèbres adjacentes sont ainsi immobilisées l'une par rapport à l'autre de façon à permettre la fusion entre ces deux vertèbres.

Cependant, un tel système est difficile à mettre en place.

US 2006/0167459 A1 décrit un système selon le préambule de la revendication 1.

Un but de l'invention est d'avoir un système plus facile à mettre en place.

A cet effet, l'invention a pour objet un système d'ostéosynthèse du type précité, les éléments de fixation comprenant:
- un premier clou destiné à être inséré dans une première vertèbre par impactage ;
- un deuxième clou sensiblement coplanaire et sensiblement parallèle au premier clou et destiné à être inséré dans une deuxième vertèbre par impactage ; et
- un pont relié rigidement aux premier et deuxième clous, caractérisé en ce que
- chaque clou est relié au pont par une tête sensiblement sphérique, ce qui permet de conserver un mouvement relatif normal entre les vertèbres dans un plan sagittal tout en les maintenant rigidement pour éviter tout déplacement hors de ce plan.

Suivant des modes particuliers de réalisation, le système d'ostéosynthèse comporte une ou plusieurs des caractéristiques suivantes, prise(s) isolément ou suivant toutes les combinaisons techniquement possibles :
- la tête de chacun des premier et second clous a une forme adaptée pour l'impactage et le dévissage du clou ;
- la tête comporte au moins une encoche de dévissage formant des surfaces de prise pour le dévissage du clou ;
- il comporte en outre un troisième clou disposé entre les premier et deuxième clous et destiné à être inséré dans une troisième vertèbre par impactage, le troisième clou étant relié aux premier et deuxième clous par le pont ;
- le pont présente une flexibilité adaptée pour autoriser un mouvement de flexion-extension entre les vertèbres qui reçoivent les clous reliés par le pont ;
- le pont a une forme courbe ;
- le pont est convexe vers l'extrémité distale des clous ;
- les clous adjacents forment entre eux un angle non nul, de préférence environ égal à 6° ;
- chaque clou est associé à une cheville d'ancrage, la cheville étant destinée à s'élargir lors de l'insertion du clou dans la cheville, chaque clou comportant des moyens d'insertion du clou dans la cheville respective par poussée axiale et de retenue axiale du clou dans la cheville, la cheville étant utile dans la fixation pédiculaire en cas de tenue incertaine au vu de l'état de l'os ;
- les moyens d'insertion et de retenue comprennent un filetage à section asymétrique agencé de telle sorte que le clou est susceptible d'être inséré dans la cheville par impactage et d'être extrait de la cheville par dévissage ;
- le filetage présente un flanc distal incliné et un flanc proximal sensiblement radial ;
- il est réalisé dans un matériau transparent aux rayons X.

L'invention sera mieux comprise à la lecture de la description qui va suivre, donnée à titre d'exemple et se référant aux dessins annexés, sur lesquels :
- la figure 1 est une vue schématique d'une section verticale d'un système d'ostéosynthèse selon l'invention en place sur deux vertèbres ;
- la figure 2 est une vue schématique d'une section horizontale du système de la figure 1 ;
- la figure 3 est une vue schématique de côté d'une cheville de fixation selon l'invention ;
- la figure 4 est une vue schématique d'une section suivant un axe IV-IV médian de la cheville de fixation de la figure 3 ;
- la figure 5 est une vue schématique de face d'un ensemble de deux clous reliés par un pont selon l'invention ;
- la figure 6 est une vue schématique agrandie analogue à la figure 5 d'un ensemble de trois clous reliés par un pont, selon l'invention ;
- la figure 7 est une vue schématique de face agrandie avec arrachement partiel d'un clou intermédiaire de l'ensemble de la figure 6 ; et
- la figure 8 est une vue schématique en perspective de deux vertèbres illustrant le positionnement d'une cage intersomatique.

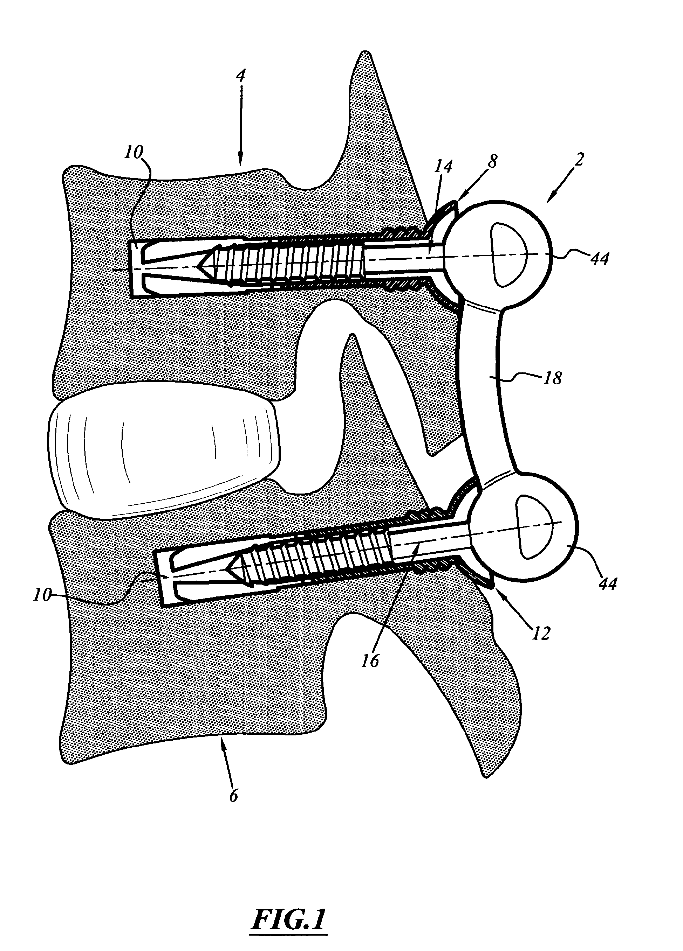

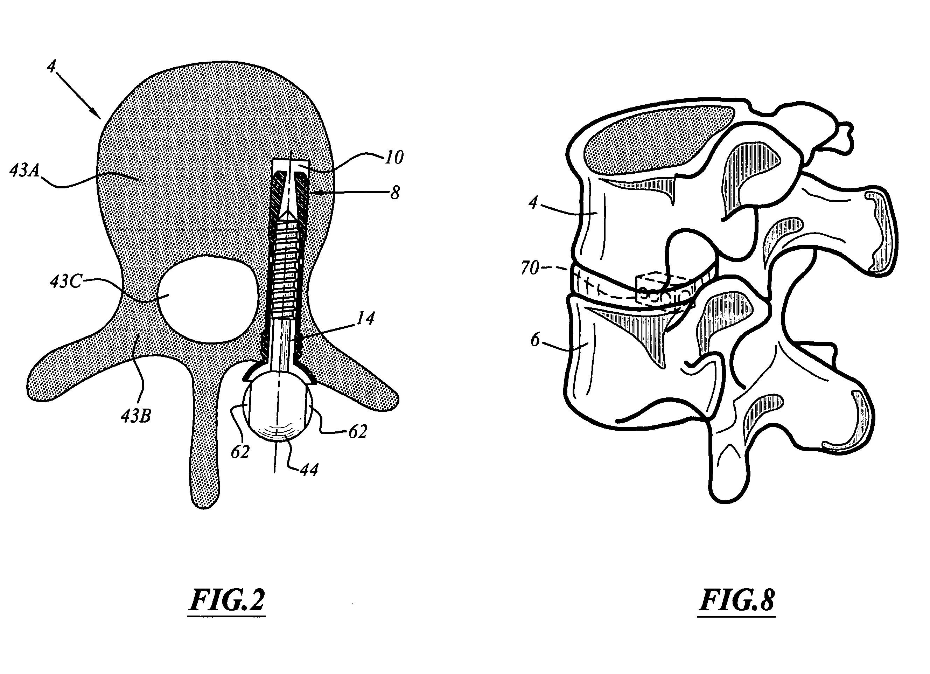

Les figures 1 et 2 illustrent un système d'ostéosynthèse par voie postérieure d'une première 4 et d'une deuxième 6 vertèbres, comprenant une première cheville 8 d'ancrage insérée dans un trou 10 ménagé dans la première vertèbre 4, une deuxième cheville 12 d'ancrage insérée dans un trou 10 ménagé dans la deuxième vertèbre 6, ainsi qu'un premier 14 et un deuxième 16 clous destinés à être insérés respectivement dans les première et deuxième chevilles 8, 12 et reliés entre eux par un pont 18 flexible.

Chaque ensemble de fixation clou-cheville 8, 14 ; 12, 16 forme un ensemble de fixation dans une vertèbre 4 ; 6.

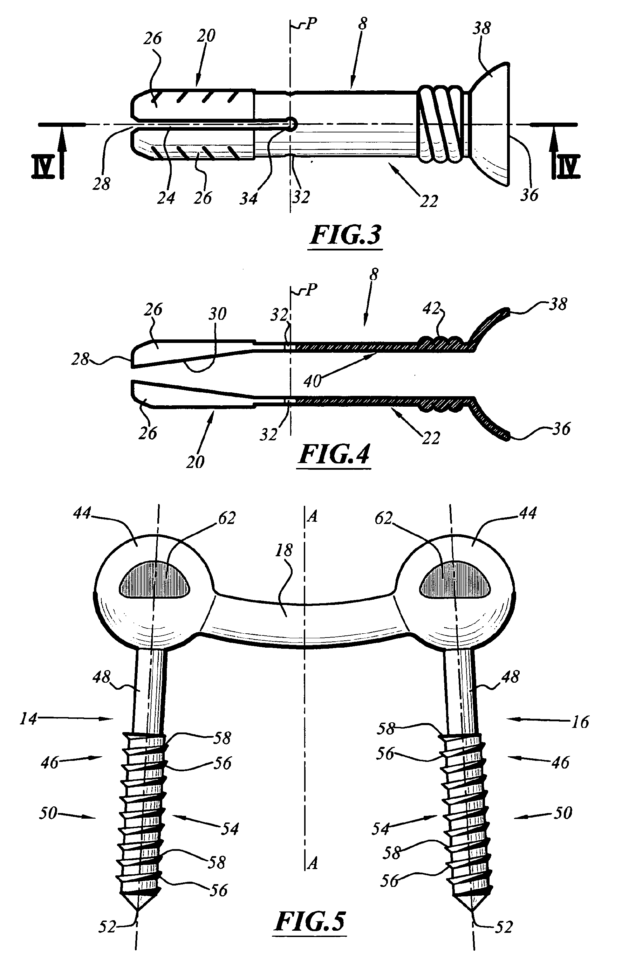

Les première et deuxième chevilles 8, 12 sont identiques et sont illustrées plus en détail sur les figures 3 et 4.

Dans tout ce qui suit, on entend par "distal" ce qui est destiné à être positionné vers l'intérieur de la vertèbre, et par "proximal" ce qui est destiné à être positionné vers l'extérieur de la vertèbre,

Chaque cheville 8, 12 est d'une seule pièce en matière plastique biocompatible et est constituée d'une partie 20 distale expansible et d'une partie 22 proximale.

La partie distale 20 comporte une fente 24 axiale coupant la partie distale 20 en deux segments 26 identiques et s'étendant jusqu'à une extrémité 28 distale de la cheville 8, 12.

Les deux segments 26 sont destinés à s'écarter radialement l'un de l'autre lors de l'insertion du clou 14, 16 dans la partie distale 20 de la cheville 8, 12. A cet effet, la partie 20 a un diamètre interne décroissant vers l'extrémité distale 28 et forme, dans l'exemple illustré sur la figure 4, une portion 30 interne tronconique.

En outre, les deux segments 26 comportent chacun un trou 32 radial, les trous 32 étant ménagés radialement dans un même plan P perpendiculaire à l'axe de la cheville. Les trous 32 favorisent l'écartement des segments 26 au niveau des trous 32 lors de l'insertion du clou 14, 16.

De la même manière, la fente 24 se termine de son côté proximal par un évidement 34 cylindrique formant deux trous de même diamètre que les trous 32. L'évidement 34 est dans le même plan axial que la fente 24 et dans le même plan P perpendiculaire à l'axe de la cheville 8, 12 que les trous 32.

La partie proximale 22 comporte, de l'extrémité 36 proximale de la cheville 8, 12 vers la partie distale 20, une portion d'entrée 38 évasée destinée à guider le clou 14, 16, puis une portion 40 à diamètre interne constant reliant la portion évasée 38 à la partie distale 20, comme illustré sur la figure 4.

La portion 40 comporte, à son extrémité proximale, un filetage 42 externe faisant saillie radialement, s'étendant par exemple sur environ 1 centimètre et permettant un pré-ancrage de la cheville 8, 12 dans la vertèbre 4, 6 avant insertion du clou 14, 16. Le filetage 42 a, dans l'exemple illustré, un profil cylindrique et un pas d'environ 1/3 cm. Un autre type de filetage 42 peut néanmoins être utilisé.

Sur le reste de la portion 40, la surface externe est lisse et cylindrique.

Comme illustré sur la figure 2, la partie distale 20 est destinée à être positionnée dans la partie intra-somatique 43A de la vertèbre 4, 6, tandis que la partie proximale 22 est destinée à être positionnée dans la partie intra-pédiculaire 43B de la vertèbre 4, 6, la partie de la portion 40 dont la surface externe est cylindrique étant destinée à longer le tunnel intra-pédiculaire 43C.

La longueur de la cheville 8, 12 est par exemple comprise entre 30 mm et 40 mm, la partie proximale 22 ayant une longueur comprise entre 20 et 25 mm, et la partie distale 20 ayant une longueur d'environ 15 mm.

Le diamètre externe de la partie distale 20 mesuré au niveau de la portion 30 est supérieur au diamètre externe de partie de la portion 40 dont la surface externe est cylindrique. Le diamètre externe de la partie distale 20 est par exemple d'environ 6 mm, tandis que la partie à surface externe cylindrique de la portion 40 a un diamètre externe d'environ 5 mm.

La figure 5 illustre les premier et deuxième clous 14, 16 ainsi que le pont 18 les reliant.

Les premier et deuxième clous 14, 16 sont identiques. Ils comportent une tête 44 en forme générale de sphère et une tige 46 qui part de la tête 44.

La tête 44 est destinée à être reçue dans la portion d'entrée 38.

La tige 46 comporte une partie 48 proximale dont la surface externe est cylindrique, et une partie 50 distale prolongeant la partie 48 jusqu'à une pointe 52 distale du clou 14, 16.

La partie distale 50 comporte un filetage 54 à section asymétrique s'étendant sur toute la longueur de la partie distale 50, jusqu'à la pointe 52. Le filetage 54 a un profil en dent de scie et comprend un flanc 56 distal incliné et un flanc 58 proximal sensiblement radial formant entre eux un angle compris entre 15 et 60°, de préférence entre 30 et 45°. Les flancs distal et proximal 56, 58 forment ainsi un profil en dent de scie triangulaire. Néanmoins, le filetage peut aussi avoir un profil trapézoïdal ou un autre profil approprié.

L'inclinaison du flanc distal 56 par rapport à l'axe du clou 14, 16 est par exemple comprise entre 15 et 75°, de préférence ent re 20 et

L'inclinaison du flanc proximal 58 par rapport à l'axe du clou 14, 16 est quant à elle par exemple comprise entre 60 et 90°, de pré férence entre 80 et 88°.

Le diamètre externe de la partie distale 50 est légèrement supérieur au diamètre interne de la partie proximale 22 de la cheville 8, 12. Le diamètre externe de la partie proximale 48 est inférieur au diamètre externe de la partie distale 50 et inférieur au diamètre interne de la partie 22.

Le filetage 54 constitue un moyen d'insertion par poussée axiale du clou 14, 16 dans la cheville 8, 12 et de retenue axiale. En effet, la faible inclinaison du flanc distal 56 permet le glissement du clou 14, 16 sur la surface interne de la cheville 8, 12, dans le sens orienté vers l'extrémité distale 28 de la cheville 8, 12. La forte inclinaison du flanc proximal 58 du filetage 54 empêche le glissement du clou 14, 16 sur la surface interne de la cheville, dans le sens orienté vers l'extrémité proximale 36 de la cheville 8, 12. Ainsi, le clou 14, 16 est propre à être inséré dans la vertèbre 4, 6 par impactage ou translation axiale sans rotation. Le clou 14, 16 est, après impactage, immobilisé en translation par la cheville 8, 12 du fait de la pression exercée par la cheville 8, 12, sur les flancs distal et proximal 56, 58. Pour l'extraction du clou 14, 16, on procède d'abord au sciage du pont 18 au niveau de la tête 44, puis au dévissage du clou 14, 16.

Le pont 18 relie les têtes 44 des premier et deuxième clous 14, 16. Il est réalisé par une tige en forme d'arc de cercle dont le rayon de courbure est d'environ 430 mm et dont l'axe est perpendiculaire au plan contenant les axes des deux clous 14, 16. Le centre de cet arc de cercle est situé du côté proximal, de sorte que le pont 18 est convexe vers le côté distal.

L'ensemble formé par le pont 18 et les deux clous 14,16 est, comme illustré sur la figure 5, symétrique par rapport à un plan médian A-A, situé à mi-distance des deux clous 14, 16 et perpendiculaire au pont 18.

Le pont 18 a une section circulaire d'environ 3 mm. En outre, le pont est relié aux deux têtes 44 de sorte que les axes des clous 14, 16 forment entre eux un angle d'environ 6° correspondant à une angulation lombaire physiologique entre deux vertèbres 4, 6.

Le pont 18 est flexible autour de sa position initiale de repos, visible sur la figure 5, essentiellement dans le plan contenant les axes des deux clous 14, 16, en raison de sa forme courbée. En revanche, le pont 18 évite toute translation horizontale (parallèlement au plan médian A-A) et rotation axiale des clous 14, 16. La tête 44 de chaque clou 14, 16 comporte deux encoches 62 parallèles à l'axe du clou et opposées permettant le serrage de la tête 44 pour faire tourner le clou 14, 16 et ainsi le dévisser.

La forme sphérique de la tête 44 assure une répartition optimale des contraintes mécaniques entre le pont 18 et les deux clous 14, 16 tout en gardant une importante capacité de déformation élastique au niveau de la jonction entre le pont 18 et la tête 44.

La flexibilité du pont 18 est telle que la force nécessaire pour écarter ou rapprocher les extrémités distales des clous 14, 16 en partant de leur position initiale de repos dans laquelle les axes des clous 14, 16 forment entre eux un angle d'environ 6° jusqu'à une position dans laquelle les axes des clous 14, 16 sont parallèles, est par exemple comprise entre 25 et 50 N.

Les deux clous 14, 16 et le pont 18 sont par exemple venus de matière et réalisés dans un matériau transparent aux rayons X comme le Cerlac ^{™}.

Le système d'ostéosynthèse 2 ne comprend pas nécessairement de cheville 8, 12. L'ensemble clou-pont 14, 16, 18 est alors par exemple fixé aux vertèbres 4, 6 à l'aide d'un ciment approprié de type connu.

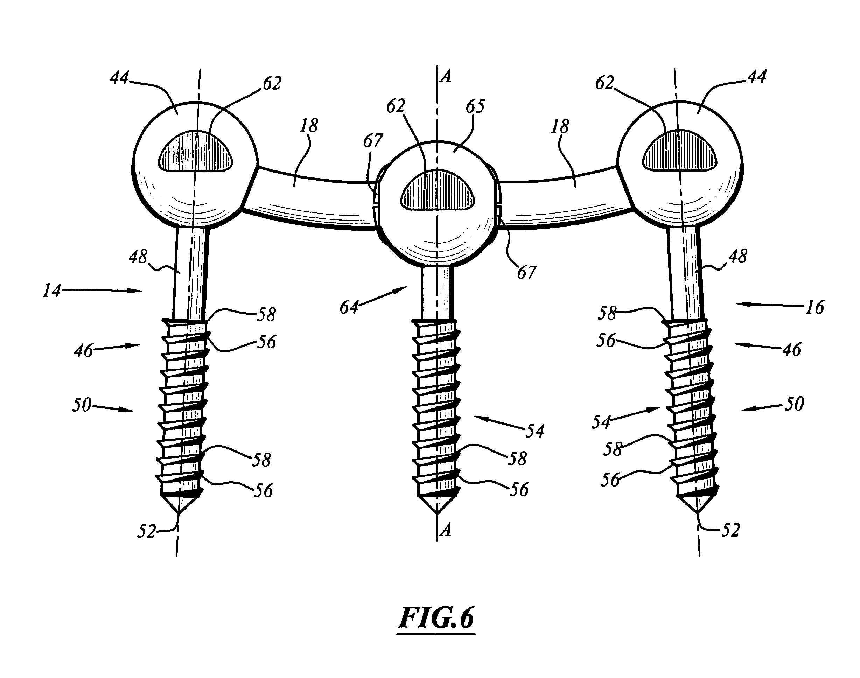

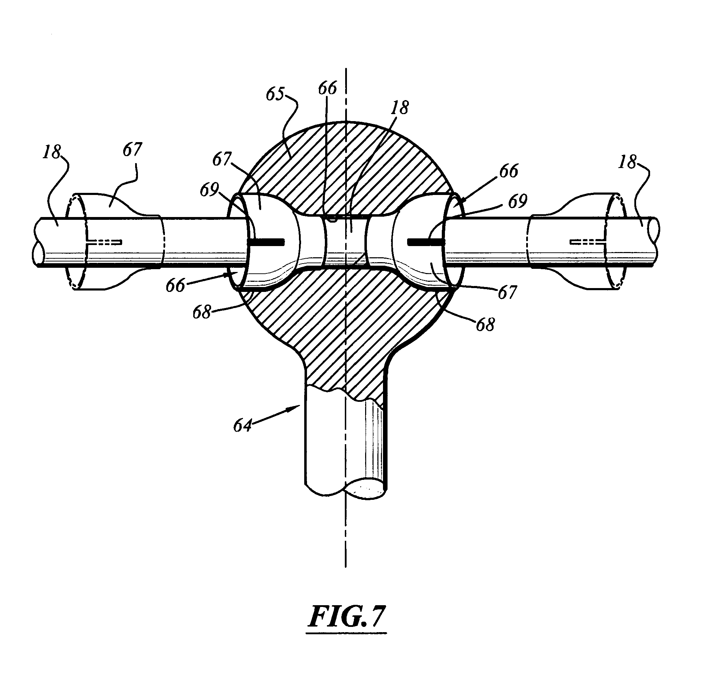

Dans un autre mode de réalisation, illustré sur les figures 6 et 7, le système d'ostéosynthèse 2 est sensiblement analogue au système 2 du premier mode de réalisation mais diffère essentiellement en ce que l'ensemble de fixation comporte trois clous 14, 16, 64 de longueur analogue, et un pont unique 18 reliant les trois clous 14, 16, 64 entre eux. Les clous 14, 16, 64 comprennent un clou intermédiaire 64 et deux clous extrêmes 14, 16.

Les clous extrêmes 14, 16 sont sensiblement identiques aux clous 14, 16 du premier mode de réalisation et forment entre eux un angle d'environ 6°. Leur tête 44 comporte un trou (non représenté) de réception du pont 18 en ajustement serré.

Le pont 18 est ainsi solidaire des clous 14, 16. Le pont 18 est analogue au pont 18 du précédent mode de réalisation mais a une longueur adaptée pour que les trois clous 14, 16, 64 soient en regard de trois vertèbres adjacentes.

Le clou 64 a une forme sensiblement identique aux clous 14, 16 mais diffère essentiellement par une tête 65 sphérique qui comporte un trou 66 la traversant (voir figure 7).

Le trou 66 reçoit le pont 18, de telle sorte que le pont 18 traverse librement la tête 65 de part et d'autre.

Le système 2 comporte en outre deux colliers 67 de fixation montés coulissants sur le pont 18, de part et d'autre du clou 64, et aptes à immobiliser le clou 64 en coulissement par rapport au pont 18.

La figure 7 illustre en trait mixte le système avant fixation du clou 64 sur le pont 18. Les colliers 67 sont alors espacés du clou 64.

Le trou 66 comporte deux surfaces 68 évasées de réception en force des colliers 67, comme illustré sur la figure 7. En effet, les surfaces 68 ont une forme complémentaire des colliers 67 mais ont un diamètre plus petit que les colliers 67 de façon à ce que la mise en place des colliers 67 dans le trou 66 se fasse en force. A cet effet, les colliers 67 comportent une fente 69 axiale permettant leur déformation.

La mise en place des colliers 67 dans le trou 66 se fait par impactage. Une fois impactés dans le trou 66, comme illustré sur les figures 6 et 7, le clou 64 est fixé sur le pont 18.

La mobilité du clou 64 par rapport au pont 18 facilite la mise en place du système 2 sur trois vertèbres 4, 6. La fixation du clou 64 par rapport au pont 18 est par exemple réalisée une fois l'insertion des clous 14, 16, 64 dans les vertèbres 4, 6 effectuée.

Ce système à trois clous permet donc d'immobiliser trois vertèbres 4, 6, ce qui est par exemple particulièrement intéressant dans les cas d'arthrodèse L5S1 avec cage intersomatique lorsque le niveau inter sus jacent L4L5 présente un niveau distal altéré.

Dans un autre mode de réalisation, le système d'ostéosynthèse 2 à trois clous 14, 16, 64 est réalisé en une seule pièce.

Dans un autre mode de réalisation illustré sur la figure 8, le système d'ostéosynthèse 2 comporte en outre une cage 70 intersomatique de type connu. Cette dernière est destinée à être insérée entre des plateaux vertébraux des première et deuxième vertèbres 4, 6, de manière à neutraliser la rotation intervertébrale et à assurer ainsi une stabilité immédiate. En outre, la cage 70 assure un effet « spacer » qui rétablit la distraction vertébrale.

La cage intersomatique 70 est de préférence munie d'un greffon osseux, de manière connue.

La flexibilité du pont 18 est choisie de manière que le système d'ostéosynthèse 2 selon l'invention autorise un faible mouvement de flexion-extension des deux vertèbres 4, 6 entre elles. En outre, il neutralise sensiblement toute translation horizontale et rotation axiale des vertèbres entre elles.

Lorsque le système comporte une ou plusieurs cages 70, l'ensemble clou-pont 14, 16, 18 maintient une certaine contrainte sur les structures osseuses et sur le greffon osseux contenu dans la ou les cages 70, grâce à la flexibilité du pont 18, avec pour corollaire l'accélération du processus d'ostéogénèse et donc de fusion des vertèbres 4, 6.

La cheville 8, 12 permet d'absorber les contraintes transmises par le clou 14, 16 à la vertèbre 4, 6, tout en augmentant l'ancrage du système 2 au niveau de la partie intra-somatique 43A de la vertèbre 4, 6. La cheville 8, 12 est en effet prévue pour s'élargir dans la partie intra-somatique 43A de la vertèbre 4, 6, qui représente la zone d'ancrage la plus efficace et la plus large dans la vertèbre 4, 6.

La cheville 8, 12 présente également un intérêt dans le cadre d'une reconstruction après désolidarisation accidentelle d'un système d'ostéosynthèse 2 ayant entraîné un élargissement du trou 10. La cheville 8, 12 permet alors l'ancrage du clou 14, 16 malgré l'élargissement du trou 10.

Enfin, la réalisation de l'ensemble clou-pont 14, 16, 18 en matériau bio composite transparent aux rayons X permet notamment une meilleure visibilité des vertèbres.

La cheville 8,12 peut être utilisée en association avec un montage d'ostéosynthèse classique métallique lorsque la qualité de l'os vertébral compromet la stabilité du montage.

La cheville 8,12 est alors utilisée en renfort de l'ostéosynthèse métallique.

## Revendications

1. Système (2) d'ostéosynthèse pour relier au moins deux vertèbres, du type comportant des éléments de fixation dans chacune des vertèbres, les éléments de fixation comprenant :
- un premier clou (14) destiné à être inséré dans une première vertèbre (4) par impactage ;
- un deuxième clou (16) sensiblement coplanaire et sensiblement parallèle au premier clou (14) et destiné à être inséré dans une deuxième vertèbre (6) par impactage ; et
- un pont (18) relié rigidement aux premier et deuxième clous (14, 16), **caractérisé en ce que** chaque clou (14, 16) est relié au pont (18) par une tête (44) sensiblement sphérique.

2. Système d'ostéosynthèse selon la revendication 1, **caractérisé en ce que** la tête (44, 45) de chacun des premier et second clous (14, 16) a une forme adaptée pour l'impactage et le dévissage du clou (14, 16).

3. Système d'ostéosynthèse selon la revendication 2, **caractérisé en ce que** la tête (44, 65) comporte au moins une encoche de dévissage (62) formant des surfaces de prise pour le dévissage du clou (14, 16).

4. Système (2) d'ostéosynthèse selon l'une quelconque des revendications précédentes, **caractérisé en ce qu'**il comporte en outre un troisième clou (64) disposé entre les premier et deuxième clous (14, 16) et destiné à être inséré dans une troisième vertèbre par impactage, le troisième clou (64) étant relié aux premier et deuxième clous (14, 16) par le pont (18).

5. Système (2) d'ostéosynthèse selon l'une quelconque des revendications précédentes, **caractérisé en ce que** le pont (18) présente une flexibilité adaptée pour autoriser un mouvement de flexion-extension entre les vertèbres (4, 6) qui reçoivent les clous (14, 16, 64) reliés par le pont (18).

6. Système (2) d'ostéosynthèse selon l'une quelconque des revendications précédentes, **caractérisé en ce que** le pont (18) a une forme courbe.

7. Système (2) d'ostéosynthèse selon la revendication 6, **caractérisé en ce que** le pont (18) est convexe vers l'extrémité distale des clous (14, 16, 64).

8. Système (2) d'ostéosynthèse selon l'une quelconque des revendications précédentes, **caractérisé en ce que** les clous (14, 16, 64) adjacents forment entre eux un angle non nul, de préférence environ égal à 6°.

9. Système (2) d'ostéosynthèse selon l'une quelconque des revendications précédentes, **caractérisé en ce que** chaque clou (14, 16, 64) est associé à une cheville (8, 12) d'ancrage, la cheville (8, 12) étant destinée à s'élargir lors de l'insertion du clou (14, 16, 64) dans la cheville (8, 12), chaque clou (14, 16, 64) comportant des moyens d'insertion du clou (14, 16, 64) dans la cheville (8, 12) respective par poussée axiale et de retenue axiale du clou (14, 16, 64) dans la cheville (8, 12).

10. Système (2) d'ostéosynthèse selon la revendication 9, **caractérisé en ce que** les moyens d'insertion et de retenue comprennent un filetage (54) à section asymétrique agencé de telle sorte que le clou (14, 16, 64) est susceptible d'être inséré dans la cheville (8, 12) par impactage et d'être extrait de la cheville (8, 12) par dévissage.

11. Système (2) d'ostéosynthèse selon la revendication 10, **caractérisé en ce que** le filetage (54) présente un flanc distal (56) incliné et un flanc proximal (58) sensiblement radial.

12. Système d'ostéosynthèse selon l'une quelconque des revendications précédentes, **caractérisé en ce qu'**il est réalisé dans un matériau transparent aux rayons X.

## Claims

1. Osteosynthesis system (2) for connecting at least two vertebrae, of the type comprising fixation elements in each of the vertebrae, the fixation elements comprising:
- a first nail (14) which is to be inserted into a first vertebra (4) by impact-insertion;
- a second nail (16) which is substantially coplanar and substantially parallel with the first nail (14) and which is to be inserted into a second vertebra (6) by impact-insertion; and
- a bridge (18) rigidly connected to the first and second nails (14, 16),
**characterized in that** each nail (14, 16) is connected to the bridge (18) by a substantially spherical head (44).

2. Osteosynthesis system according to claim 1, **characterized in that** the head (44, 45) of each of the first and second nails (14, 16) has a shape suitable for the impact-insertion and the unscrewing of the nail (14, 16).

3. Osteosynthesis system according to claim 2, **characterized in that** the head (44, 65) comprises at least one unscrewing notch (62) forming engagement surfaces for the unscrewing of the nail (14, 16).

4. Osteosynthesis system (2) according to any one of the preceding claims, **characterized in that** it also comprises a third nail (64) which is located between the first and second nails (14, 16) and which is to be inserted into a third vertebra by impact-insertion, the third nail (64) being connected to the first and second nails (14, 16) by the bridge (18).

5. Osteosynthesis system (2) according to any one of the preceding claims, **characterized in that** the bridge (18) has a flexibility suitable for permitting a bending-extension movement between the vertebrae (4, 6) which receive the nails (14, 16, 64) connected by the bridge (18).

6. Osteosynthesis system (2) according to any one of the preceding claims, **characterized in that** the bridge (18) has a curved shape.

7. Osteosynthesis system (2) according to claim 6, **characterized in that** the bridge (18) is convex towards the distal end of the nails (14, 16, 64).

8. Osteosynthesis system (2) according to any one of the preceding claims, **characterized in that** the adjacent nails (14, 16, 64) form with each other a non-zero angle, preferably approximately equal to 6°.

9. Osteosynthesis system (2) according to any one of the preceding claims, **characterized in that** each nail (14, 16, 64) is associated with an anchoring dowel (8, 12), the purpose of the dowel (8, 12) being to expand during the insertion of the nail (14, 16, 64) into the dowel (8, 12), each nail (14, 16, 64) comprising means for the insertion of the nail (14, 16, 64) into the respective dowel (8, 12) by axial thrust and for the axial retention of the nail (14, 16, 64) in the dowel (8, 12).

10. Osteosynthesis system (2) according to claim 9, **characterized in that** the insertion and retention means comprise a thread (54) which has an asymmetrical cross-section and which is in a form such that the nail (14, 16, 64) is capable of being inserted into the dowel (8, 12) by impact-insertion and of being removed from the dowel (8, 12) by unscrewing.

11. Osteosynthesis system (2) according to claim 10, **characterized in that** the thread (54) has an inclined distal flank (56) and a substantially radial proximal flank (58).

12. Osteosynthesis system according to any one of the preceding claims, **characterized in that** it is produced from a material transparent to X-rays.

## Patentansprüche

1. Osteosynthesesystem (2) zur Verbindung von mindestens zwei Wirbeln, des Typs, der Fixierelemente in jedem der Wirbel umfasst, wobei die Fixierelemente folgendes umfassen:
- einen ersten Nagel (14), der dazu bestimmt ist, in einen ersten Wirbel (4) durch Einschlagen eingeführt zu werden;
- einen zweiten Nagel (16), der im Wesentlichen koplanar und im Wesentlichen parallel zu dem ersten Nagel (14) ist und dazu bestimmt ist, in einen zweiten Wirbel (6) durch Einsschlagen eingeführt zu werden; und
- eine Brücke (18), die starr mit dem ersten und dem zweiten Nagel (14, 16) verbunden ist,
**dadurch gekennzeichnet, dass** jeder Nagel (14, 16) mit der Brücke (18) über einen im Wesentlichen kugeligen Kopf (44) verbunden ist.

2. Osteosynthesesystem nach Anspruch 1, **dadurch gekennzeichnet, dass** der Kopf (44, 45) von jeweils dem ersten und dem zweiten Nagel (14, 16) eine Form aufweist, die zum Einschlagen und zum Herausschrauben des Nagels (14, 16) ausgelegt ist.

3. Osteosynthesesystem nach Anspruch 2, **dadurch gekennzeichnet, dass** der Kopf (44, 45) mindestens eine Kerbe zum Herausschrauben (62) umfasst, die Greifflächen zum Herausschrauben des Nagels (14, 16) bildet.

4. Osteosynthesesystem (2) nach einem der vorhergehenden Ansprüche, **dadurch gekennzeichnet, dass** es weiterhin einen dritten Nagel (64) umfasst, der zwischen dem ersten Nagel und dem zweiten Nagel (14, 16) angeordnet ist und dazu bestimmt ist, in einen dritten Wirbel durch Einschlagen eingeführt zu werden, wobei der dritte Nagel (64) mit dem ersten und dem zweiten Nagel (14, 16) durch die Brücke (18) verbunden ist.

5. Osteosynthesesystem (2) nach einem der vorhergehenden Ansprüche, **dadurch gekennzeichnet, dass** die Brücke (18) eine Biegsamkeit aufweist, die ausgelegt ist, um eine Biege-Dehn-Bewegung zwischen den Wirbeln (4, 6), die die durch die Brücke (18) verbundenen Nägel (14, 16, 64) aufnehmen, zuzulassen.

6. Osteosynthesesystem (2) nach einem der vorhergehenden Ansprüche, **dadurch gekennzeichnet, dass** die Brücke (18) eine gekrümmte Form aufweist.

7. Osteosynthesesystem (2) nach Anspruch 6, **dadurch gekennzeichnet, dass** die Brücke (18) zum distalen Ende der Nägel (14, 16, 64) konvex ist.

8. Osteosynthesesystem (2) nach einem der vorhergehenden Ansprüche, **dadurch gekennzeichnet, dass** die benachbarten Nägel (14, 16, 64) miteinander einen Winkel von nicht Null, vorzugsweise von etwa gleich 6 ° bilden.

9. Osteosynthesesystem (2) nach einem der vorhergehenden Ansprüche, **dadurch gekennzeichnet, dass** jedem Nagel (14, 16, 64) ein Verankerungsdübel (8, 12) beigefügt ist, wobei der Dübel (8, 12) dazu bestimmt ist, sich bei Einführung des Nagels (14, 16, 64) in den Dübel (8, 12) zu weiten, wobei jeder Nagel (14, 16, 64) Mittel zur Insertion des Nagels (14, 16, 64) in den jeweiligen Dübel (8, 12) durch axiale Längskraft und zur axialen Retention des Nagels (14, 16, 64) in dem Dübel (8, 12) umfasst.

10. Osteosynthesesystem (2) nach Anspruch 9, **dadurch gekennzeichnet, dass** die Insertions- und Retentionsmittel ein Gewinde (54) mit asymmetrischem Querschnitt umfassen, das so gearbeitet ist, dass der Nagel (14, 16, 64) dazu geeignet ist, in den Dübel (8, 12) durch Einschlagen eingeführt und durch Herausschrauben aus dem Dübel (8, 12) herausgezogen zu werden.

11. Osteosynthesesystem (2) nach Anspruch 10, **dadurch gekennzeichnet, dass** das Gewinde (54) eine geneigte distale Seitenfläche (56) und eine im Wesentlichen radiale proximale Seitenfläche (58) aufweist.

12. Osteosynthesesystem (2) nach einem der vorhergehenden Ansprüche, **dadurch gekennzeichnet, dass** es aus einem für Röntgenstrahlen transparenten Material realisiert ist.
